# EUROPEAN PATENT SPECIFICATION

(11) **EP 3 110 861 B1**
(45) Date of publication and mention of the grant of the patent: **07.11.2018**
(21) Application number: 15704020.5
(22) Date of filing: 13.02.2015
(51) Int. Cl.: C08F 220/54, C08F 2/04

(54) **A POLYMER SUITABLE FOR USE IN HAIR STYLING**
POLYMER ZUR VERWENDUNG BEI DER HAARFORMUNG
POLYMÈRE APPROPRIÉ À UN USAGE DANS LE COIFFAGE

(30) Priority: 26.02.2014 EP 14156706
(43) Date of publication of application: 04.01.2017
(73) Proprietor: BASF SE, 67056 Ludwigshafen am Rhein (DE)
(72) Inventor: GRAHAM, David, 40213 Düsseldorf (DE); SEELIG, Bianca, 50937 Köln (DE); MACK, Sandra, 41352 Korschenbroich (DE); TONHAUSER, Christoph, 67069 Ludwigshafen (DE); HAAKE, Hans-Martin, 40699 Erkrath (DE); WENDEL, Volker, 40589 Düsseldorf (DE); GRUND, Patricia, 40789 Monheim (DE); HAUKE, Martin, 67071 Ludwigshafen (DE); JECK, Sylvia, 67434 Neustadt (DE); SCHRÖDER, Björn, 40227 Düsseldorf (DE)
(74) Representative: BASF IP Association
(86) International application number: PCT/EP2015/053066
(87) International publication number: WO 2015/128204

(56) References cited:
- EP-A1- 0 259 851
- EP-A2- 0 037 378
- GB-A- 862 490
- US-B1- 6 503 479

## Description

The present invention relates to a method for making a copolymer comprising polymerizing the monomers in a solution comprising the monomers and a solvent, wherein the solvent comprises water and an alcohol. Furthermore, the present invention relates to the copolymer obtainable by this method ("the copolymer according to the present invention") and to the use of the copolymer according to the present invention for styling or for fixing human hair. Furthermore, the present invention relates to the use of a more generally defined copolymer for styling or for fixing human hair, and for providing high gloss to the hair that is styled or fixed.

Polymers used in hair cosmetics, especially those used for hair styling, can be divided in different classes depending on their functionalities. The non-ionic polymers, anionic polymers, anionic (ampholytic) polymers as well as cationic (ampholytic) polymers are known in the state of the art and are available in the market. Water-soluble anionic polymers can be used for the styling of hair. Example products are Gantrez® types (ISP), Resyn® types (Akzo Nobel) and Luvimer®/Ultrahold® types (BASF). In the state of the art many methods of preparing said polymers are described, from solution polymerization to emulsion, suspension, and precipitation polymerization. Anionic polymers in particular can be prepared using a wide variety of methods.

US 3 112 296 discloses acrylic polymers that are useful as hair spray resins. The polymers disclosed are suitable for use as hair fixing formulations in e.g. air-pump sprays or aerosol cans. Among the particularly useful acrylic polymers is a terpolymer comprising acrylic acid (AA), ethyl acrylate (EA) and t-butylacrylamide (tBAM). In Example 1 of US Patent 3,112,296, for instance, the terpolymer AA-EA-tBAM is made by copolymerizing ethyl acrylate and t-butylacrylamide and then partially saponifying the ethyl acrylate moieties in the polymer chain to give acrylic acid moieties. According to EP 0 037 378 A2 this method results in a terpolymer having less than the ideal balance of water solubility and curl retention properties, probably due to an uneven distribution of carboxylate groups down the polymer chain. According to the claims of US 3,112,296 a preferred polymer comprises about 41.6 % by weight EA, 16.6 % by weight AA and 41.6 tBAM.

EP 0 037 378 A2 discloses a terpolymer suitable for use as hair styling polymer (hair fixing polymer). The terpolymer comprises (in % by weight) 46-56 % tBAM, 37-45 % EA and 6-8 % AA. The terpolymer is made by polymerizing the monomers in a solution of ethanol or an ethanol-water-mixture. It is disclosed that polymer films can be obtained which are transparent, glossy, flexible and strong.

EP 0 259 851 A1 discloses a terpolymer suitable for use as hair styling polymer (hair fixing polymer). The terpolymer comprises (in % by weight) 50-56 % tBAM, 37-45 % EA and 6-9 % AA. The terpolymer is made by polymerizing the monomers in the form of an emulsion in water.
Terpolymers comprising tBAM, EA and AA are commercially available. BASF (Ludwigshafen, Germany) provides Ultrahold® Strong, which is a terpolymer comprising tBAM, EA and AA and which can be used in hair-setting preparations based on alcohol and water.

Furthermore anionic polymers can be found in a variety of cosmetic compositions, often being used as film forming or fixative components for hair, skin and decorative cosmetics. WO 2011/000711 discloses reduced VOC hairsprays in combination with a propellant and a specific valve. WO 2008/041202 discloses Wax styling flakes. EP 1 813 265 A discloses the treatment of keratin with a formulation containing a fixative and at least one polyol. WO 2006/018328 discloses compact hairsprays using concentrated fixatives and special valves. Hair styling (hair fixing) polymers are required to result in polymer films having high bending stiffness, high curl retention and to result in hair with high shine (gloss).

US 6,503,479 describes the use of Ultrahold® Strong for fixing or styling hair. This polymer includes 10 % acrylic acid as disclosed in WO 94/23696.

One problem underlying the present invention is to provide a polymer suitable for use in hair styling which provides for high bending stiffness, high curl retention and which results in hair with high shine.

This problem is solved by the copolymer according to the present invention which is defined in the following paragraphs in the product by process format. Therefore, a first embodiment of the present invention is a method for making the copolymer according to the present invention as defined in the following paragraphs.

### The method for making the copolymer according to the present invention

One embodiment of the present invention is a method for making a copolymer, wherein the copolymer comprises as monomers, in polymerized form,

45 to 55 % by weight of an unsaturated amide of the following structure 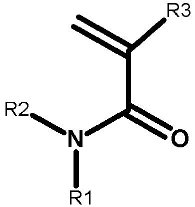 wherein
R1 is selected from the group consisting of H and C1-C8 alkyl,
R2 is selected from the group consisting of H and C1-C8 alkyl,
R3 is selected from the group consisting of H and C1-C8 alkyl,
35 - 45 % by weight of an acrylic acid ester of the following structure 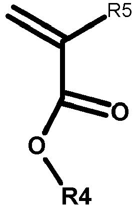
wherein
R4 is selected from the group consisting of C1-C12 alkyl,
R5 is selected from the group consisting of H and C1-C12 alkyl,
9 to 15 % by weight of an acidic monomer comprising a C=C double bond and a COOH group and
0 to 10, preferably 0 to 5, more preferably 0 to 2, % by weight of at least one further monomer having at least one C=C double bond,
wherein the acidic monomer is optionally partially or completely neutralized with a neutralizing agent, wherein the neutralizing agent is preferably selected from the group consisting of triethanolamine, AMP (2-amine-2-jmethyl-1-propanol), sodium hydroxyde, potassium hydroxyde and mixtures thereof,
said method comprising
polymerizing the monomers in a solution comprising the monomers and a solvent,
wherein the solvent comprises water and an alcohol selected from the group consisting of ethanol, isopropanol, n-butanol, tert-butanol, pentanol, pentanediol and mixtures thereof.

In the unsaturated amide of the following structure 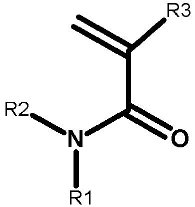
R1 is selected from the group consisting of H, C1-C8 alkyl,
preferably R1 is selected from the group consisting of isopropyl, isobutyl and tert-butyl, more preferably R1 is tert-butyl (also referred to as t-butyl),
R2 is selected from the group consisting of H, C1-C8 alkyl,
preferably R2 is selected from the group consisting of H, isopropyl, isobutyl and tert-butyl, more preferably R2 is H,
R3 is selected from the group consisting of H and C1-C8 alkyl,
preferably R3 is selected from the group consisting of H, isopropyl, isobutyl and tert-butyl, more preferably R3 is H.

The amount of this monomer in the copolymer according to the present invention is 45 to 55 % by weight, preferably 46 to 54 % by weight, more preferably 47 to 54 % by weight, even more preferably 48 to 53 % by weight, even more preferably 49 to 53 % by weight.

In the acrylic acid ester of the following structure 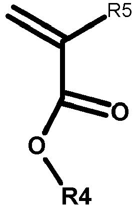
R4 is selected from the group consisting of C1-C12 alkyl,
preferably R4 is selected from the group consisting of ethyl, isopropyl, isobutyl and tert-butyl more preferably R4 is ethyl,
R5 is selected from the group consisting of H and C1-C12 alkyl,
preferably R5 is selected from the group consisting of H, ethyl, isopropyl, isobutyl and tert-butyl more preferably R5 is H.

The amount of this monomer in the copolymer according to the present invention is 35 to 45 % by weight, preferably 36 to 44 % by weight, more preferably 37 to 43 % by weight, even more preferably 37 to 42 % by weight, even more preferably 37 to 42 % by weight.

The acidic monomer comprising a C=C double bond and a COOH group is preferably selected from the group consisting of acrylic acid and methacrylic acid, more preferably it is acrylic acid.

The amount of this monomer in the copolymer according to the present invention is 9 to 15 % by weight, preferably 9 to 44 % by weight, more preferably 9 to 13 % by weight, even more preferably 9 to 12 % by weight, even more preferably 10 to 11 % by weight.

More specific embodiments of the method for making a copolymer according to the present invention are listed in the following paragraphs.
1. A method for making a copolymer,
   wherein the copolymer comprises as monomers, in polymerized form,
   45 to 55 % by weight of t-butylacrylamide,
   35 to 45 % by weight of ethyl acrylate,
   9 to 15 % by weight of acrylic acid (calculated as free acrylic acid), and
   0 to 10, preferably 0 to 5, more preferably 0 to 2, % by weight of at least one further monomer having at least one C=C double bond,
   wherein the acrylic acid is optionally partially or completely neutralized with a neutralizing agent, wherein the neutralizing agent is preferably selected from the group consisting of triethanolamine, AMP (2-amine-2-jmethyl-1-propanol), sodium hydroxyde, potassium hydroxyde and mixtures thereof,
   said method comprising
   polymerizing the monomers in a solution comprising the monomers and a solvent,
   wherein the solvent comprises water and an alcohol selected from the group consisting of ethanol, isopropanol, n-butanol, tert-butanol, pentanol, pentanediol and mixtures thereof.

The method of the previous paragraph is hereinafter referred to as "embodiment 1".
2. The method of embodiment 1
   wherein the copolymer comprises as monomers, in polymerized form,
   46 to 54 % by weight of t-butylacrylamide,
   36 to 44 % by weight of ethyl acrylate,
   9 to 14 % by weight of acrylic acid (calculated as free acrylic acid), and
   0 to 10, preferably 0 to 5, more preferably 0 to 2, % by weight of at least one further monomer having at least one C=C double bond.
3. The method of embodiment 1,
   wherein the copolymer comprises as monomers, in polymerized form,
   47 to 54 % by weight of t-butylacrylamide,
   37 to 43 % by weight of ethyl acrylate,
   9 to 13 % by weight of acrylic acid (calculated as free acrylic acid), and
   0 to 10, preferably 0 to 5, more preferably 0 to 2, % by weight of at least one further monomer having at least one C=C double bond.
4. The method of embodiment 1,
   wherein the copolymer comprises as monomers, in polymerized form,
   48 to 53 % by weight of t-butylacrylamide,
   37 to 42 % by weight of ethyl acrylate,
   9 to 12 % by weight of acrylic acid (calculated as free acrylic acid), and
   0 to 10, preferably 0 to 5, more preferably 0 to 2, % by weight of at least one further monomer having at least one C=C double bond.
5. The method of embodiment 1,
   wherein the copolymer comprises as monomers, in polymerized form,
   48 to 53 % by weight of t-butylacrylamide,
   37 to 41 % by weight of ethyl acrylate,
   10 to 12 % by weight of acrylic acid (calculated as free acrylic acid), and
   0 to 10, preferably 0 to 5, more preferably 0 to 2, % by weight of at least one further monomer having at least one C=C double bond.
6. The method of embodiment 1,
   wherein the copolymer comprises as monomers, in polymerized form,
   49 to 53 % by weight of t-butylacrylamide,
   37 to 41 % by weight of ethyl acrylate,
   10 to 11 % by weight of acrylic acid (calculated as free acrylic acid), and
   0 to 5, preferably 0 to 2, % by weight of at least one further monomer having at least one
   C=C double bond.
7. The method according to the present invention or according to any of the embodiments 1 to 6
   wherein the solvent comprises
   20 to 70, preferably 20 to 50, more preferably 25 to 35 % by weight of water, and
   30 to 80, preferably 50 to 80, more preferably 65 to 75 % by weight of an alcohol selected from the group consisting of ethanol, isopropanol, n-butanol, tert-butanol, pentanol, pentanediol and mixtures thereof.
8. The method according to embodiment 7, wherein the alcohol is ethanol.

### The copolymer according to the present invention

A further embodiment of the present invention is a copolymer obtainable by the method for making the copolymer according to the present invention (including the specific embodiments described in the previous paragraphs which result in specific embodiments of the copolymer according to the present invention).

This copolymer is obtainable in the solvent that is used in the method for making the copolymer, if this method is used. This solution can be used for making hair care products. Alternatively the copolymer may be isolated and then used for making hair care products.

### The use of the copolymer according to the present invention

A further embodiment of the present invention is the use of the copolymer according to the present invention for styling or for fixing human hair (including the specific embodiments described in the previous paragraphs which result in specific embodiments of the use of the copolymer according to the present invention).
A more specific use is the use of the copolymer according to the present invention for styling or for fixing human hair, and for providing high gloss to the hair that is styled or fixed (including the specific embodiments described in the previous paragraphs which result in specific embodiments of this use of the copolymer according to the present invention).

The following paragraphs describe how the polymer according to the present invention (also referred to as "styling polymer") can be applied to hair. % means % by weight.
The polymer according to the present invention can be formulated into hair styling products based on hair spray compositions, hair spray, hair gel, mousse or wax.

Typical hair spray formulations can be a composition of styling polymer 1-7%, ethanol, a neutralizing agent (AMP (2-Amino-2-Methyl-1-Propanol), NaOH, KOH, or combinations thereof) and a propellant (DME (dimethyl ether), propane, butane, isobutane, HFC-152a or combinations thereof). Depending on the VOC (volatile organic compounds) formulations with 55, 80, 90% or higher amounts of VOCs the rest of the formulation can be water or EtOH.

Typical gel formulations can be a composition of styling polymer, from 0,5-7%, possibly in combination with other, commercially available styling polymers such as poly vinyl pyrrolidone PVP K30, PVP K90, Luviset® Clear, and Luviset® One (also varying between 0-5%). The styling polymer can be neutralized with e.g. triethanolamine, AMP (2-Amino-2-Methyl-1-Propanol), NaOH or KOH. The thickening component of the gel formulation can be chosen from the pure polyacrylic acid based thickeners, e. g. Carbopol® 980, or Cosmedia® CPlus, or from hydrophobically modified alkali swellable emulsions such as Luvigel® Fit, or Acculyn® 22 and such polymers (from 0,5-5%). The aforementioned thickening polymers can be neutralized using the same neutralizing agents as mentioned previously.

Typical mousse formulations may contain one or more styling polymer (1-9%), which can additionally be combined with a variety of standard styling polymers such as Polyquaternium® 11, Luviquat® Supreme, Luviquat® hold, Luviflex® soft, Luvikol® PVP or Luviskol® VP/VA types. Furthermore mousse formulations can contain water (40-95%), a neutralizing agent chosen from AMP, triethanolamine, NaOH or KOH from (0,01-5%), solubilisers chosen from hydrogenated oils such as PEG-40 Hydrogenated castor oil (0,0-3,0%), active ingredients such as UV filters or sensory improvers e.g. panthenol can also be included in formulations (0,0-1,5%). Emulsifiers of the O/W type such as Ceteareth®-25 (Cremophor® AT25) and similar structures are also commonly found in mousse formulations (0,1-2,5%). Depending on the desired final consistency of the mousse a rheology modifier can also be included in the final formulation Hydroxyethylcelluloses and their derivatives are commonly used for this task, e.g. Natrosol® 250HR (0,0-1,5%), finally a propellant is required, this can be chosen from propane/butane, or isobutene and is generally present in the range from 10-60%.

A suitable initiator for making the polymer according to the present invention can be any initiator. It can be chosen from tert.-butyl peroctoate, 2,2'-Azobis(2-amidonopropane) dihydrochloride (V50); 2,2'-Azobis(2,4-dimethylvaleronitrile) (V65); tert.-butylperoxy pivalate; tert.-butylperoxy butyrate; tert.-butylperoxy-2-ethylhexanoate; sodium peroxodisulphate (NaPS); ammonium peroxodisulphate (APS) and 2,5-Dimethyl-2,5-di(tert-butylperoxy) hexane.

Another embodiment of the present invention is a formulation of the polymer according to the present invention in combination with a propellant and other commonly used cosmetic ingredients. Propellants can be selected from the group consisting of DME (dimethyl ether), propane/butane, isobutene, fluorocarbon 152a, and combinations thereof. The propellant or propellant combination can make up from 15-48 % by weight of the formulation. Ethanol, water and other cosmetically acceptable components can be present in the formulation in such quantities to enable the preparation of a final cosmetic aerosol with a widely accepted volatile organic (VOC) content of 52 - 99 % by weight.

### Examples

% means % by weight unless defined differently.
AS or a s means active substance.

### Synthesis of Polymer A

Polymer A is a copolymer consisting of tert-butylacrylamide (51%), ethyl acrylate (39%), acrylic acid (10%) delivered in a final product form of 30% polymer in a hydroalcoholic solution composed of 70% ethanol and 30% water. Therefore the overall composition of the final product is Polymer A: 30%, ethanol: 49%, water: 21%.

A solution polymerization was carried out in a solvent mixture of ethanol and water (70% ethanol, 30% water) under an inert atmosphere. A reactor was precharged with 5% of the monomer feed (tBAM/EA/AS) and an initiator (*tert*-butylperoctoate) feed and heated up to a temperature of 70°C. The residual monomer solution (in EtOH/H₂O) was added to the reaction mixture in 2 h and the initiator solution in 3 h. The mixture was stirred for 2 h at 70 °C prior to the addition of the second initiator (*tert*-butylperoctoate) in 0.5 h. Finally, the reaction mixture was stirred for up to 6h at 85 °C to obtain the product, Polymer A.

### Rate of Dissolution

The rate at which a polymer can be formulated is of critical importance for customers, as the more readily a polymer can be formulated leads to savings not only in time but also to a reduction in energy costs and a more sustainable process.
Polymer A is compared to Polymer B. Polymer B has the same monomer composition as Polymer B but is obtained by polymerizing an emulsion of the monomers in water (similarly as described in EP 0 259 851 A1) and provided in the form of beadlets.

| Polymer | End solution goal | Method | Time to complete dissolution |
|---|---|---|---|
| Polymer B | 10% a.s in EtOH | Required quantity of EtOH was precharged in a wide-necked flask equipped with a magnetic stirrer, Polymer B was introduced in sufficient quantity to create a 10% a.s solution | 6 minutes |
| Polymer A | 10% a.s in EtOH | Required quantity of EtOH was precharged in a wide-necked flask equipped with a magnetic stirrer, Polymer A was introduced in sufficient quantity to create a 10% a.s solution | 1 minute |

### Bending Stiffness/Curl Retention

Bending stiffness measured via the 3 point method is an important indication of how hard a setting polymer will be in the hair and the level of hold which said polymer can deliver. Curl retention is a measure of how resistant the polymer film is to humidity and its ability to hold a curl in form over a fixed time period under high humidity conditions.

The process of preparing hair samples and measuring via the 3-point bending method is as follows.

A 5% a.s solution of a polymer is prepared as an ethanolic solution, 0,7g of the said solution is then applied to a hair tress with 24cm free hair length. The hair tress is then suspended in a rack to dry at 21°C and 65% relative humidity overnight in a climate chamber. The dried polymer treated hair tress is then measured on a texture analyzer (model XT plus from the company Stable Microsystems) by laying the hair tress on a frame which has two rolls separated by 9cm on which to symmetrically rest the treated hair tress. A round edged stamp is then driven from the upper side of the hair tress exactly in the middle of the two rolls on which the hair tress is resting. The strand is bent by 40mm through this action, completely fracturing the polymer film. The force required for the breaking of the polymer film is then determined in N by means of a load cell.

The curl retention of the different samples was determined as follows.

A 15cm clean hair tress was dipped three times in distilled water until it was completely soaked, said hair tress was then wrapped around a roller so as to give a tightly packed curled form. The hair tress (still) on the roller was dried in a vacuum oven for 3 hours at 70°C. After the drying period the hair tress was carefully removed from the roller resulting in a water wave curl form. This curl was then suspended from a rotating device and sprayed with a chosen hairspray formulation for a fixed amount of time based on the rate of spraying until a fixed quantity of formulation (3g) has been sprayed onto the hair. The sprayed hair tress is then allowed to dry at room temperature for one hour. Following the drying period the sprayed hair tress is suspended in a rack and the initial length of the curled hair tress is measured. The rack is then placed in a climate chamber at 25°C and 90% relative humidity.

The length of the curl is then recorded against time, with measurements being carried out after 5 hours and 24 hours. The curl retention is then expressed as a percentage, the higher the percentage the nearer the end length of the curl is to the starting length and thus the better the curl retention.

**Table 1: Comparison of Bending Stiffness and Curl Retention of Polymer A versus benchmarks**

| Material (3%) | Bending Stiffness (cN) | Curl Retention 25°C 90% relative humidity | |
|---|---|---|---|
| | | 5h | 24h |
| Polymer A | 149 | 72 | 72 |
| Benchmark 1 | 113 | 70 | 68 |
| Benchmark 2 | 119 | 73 | 71 |
| Benchmark 3 | 111 | 50 | 47 |

Benchmark 1 is a tert-Butylacrylamide/Acrylates copolymer (Ultrahold® Strong by BASF)
Benchmark 2 is an Octylacrylamide/Acrylates/Butylaminomethacrylate copolymer
Benchmark 3 is a VA/Crotonates/Vinyl Neodecanoate copolymer

Polymer A prepared via the solution polymerization method has been shown to have benefits in both stiffness and curl retention against polymers which are recognized as market benchmarks in the area of styling.

### Shine

Polymer A has been shown to provide an improvement in perceived shine compared with market benchmarks, in an independent blind study of hairspray formulations sprayed onto hair tresses. The test was constructed as follows, for the test two hair tresses per product were used, the hair tresses having a round profile. Each of the hair tresses was cleaned and formed around a roller as with the previously described curl retention to create a curl form. The curl form was then treated with a fixed amount of hairspray. After drying the perceived gloss of the hair tresses was assessed by a trained panel under a standardized light source in a darkened room, the comparison of gloss was always made pairwise.

| Comparison | | Results (Preference with respect to shine for a particular hair strand treated with hairspray) Panel of 25 persons | | | | |
|---|---|---|---|---|---|---|
| 1st | 2nd | 1^{st} preferred | | 2^{nd} preferred | | No preference |
| | | n | % | n | % | n |
| Hairspray 1 | Hairspray 2 | 0 | 0 | 25 | 100 | 0 |
| Hairspray 1 | Hairspray 3 | 0 | 0 | 25 | 100 | 0 |
| Hairspray 2 | Hairspray 3 | 1 | 4 | 22 | 88 | 2 |

Hairspray 1 is a market formulation containing
Octylacrylamide/Acrylates/Butylaminomethacrylate copolymer..
Hairspray 2 is a market formulation containing
Octylacrylamide/Acrylates/Butylaminomethacrylate copolymer combined with a VP/VA copolymer
Hairspray 3 is a market relevant formulation containing Polymer A.

The result clearly show that the market relevant formulation containing polymer A is preferred with respect to the aspect shine compared with two other market formulations which are claimed to improve shine when used.

### Further examples

Polymer A made as described before.
Polymer A (w) made as described before and called Polymer B.
The other polymers made analogously.

| Product (5%) | tBAM/EA/ AS | Polymerisation Method | Process Solvent(s) | % Solvent( s) | Bending Stiffness (cN) [+/-] | Refractive Index | Curl Retention after 24h (%) [+/-] |
|---|---|---|---|---|---|---|---|
| Polymer A | 51/39/10 | Solution | EtOH/H₂O | 70/30 | 539 [30] | 1,3760 | 69 [2] |
| Polymer A (w) | 51/39/10 | Suspension | H₂O | 100 | 525 [29] | 1,3735 | 70 [3] |
| Polymer A 8 (w) | 51/41/8 | Suspension | H₂O | 100 | 349 [28] | 1,3735 | 71 [3] |
| Polymer A (i) | 51/39/10 | Solution | ⁱPrOH | 100 | 204 [18] | 1,3785 | 49 [11] |
| Polymer A (i/w) | 51/39/10 | Solution | ⁱPrOH | 70/30 | 240 [23] | 1,3775 | 53 [13] |

### Conclusion:

The solution polymerisation process offers significantly improved gloss/shine indicated by the higher refractive indices for the polymers made by solution polymerization in comparison to polymers of the same composition prepared via a heterogeneous polymerisation process (suspension). The results also demonstrate the importance of solvent choice when performing the solution polymerisation as the same composition prepared in EtOH/H₂O mixture (Polymer A), in ⁱPrOH (Polymer A (i)), and ⁱPrOH/H₂O (Polymer A (i/w)) shows markedly different performance in the bending stiffness test (an important parameter fpr styling polymers).

Polymer A prepared in a mixture of EtOH and H₂O shows a high bending stiffness which is indicative of a strong hold, polymers A(i) and A(i/w) show reduced bending stiffness which would be less desirable in the chosen cosmetic applications. The process in EtOH/H₂O provides the optimal combination of results in comparison to the heterogeneous polymerisation process in terms of gloss and bending stiffness.

The differences in bending stiffness and curl retention between the identical polymers (Polymer A in the "Examples" and in the "Further Examples" and Polymer B in the "Examples" and in the "Further Examples" (there called Polymer A (w)) are due to the fact that different types of hair tresses were used in the "Examples" and in the "Further Examples". This means that the results of bending stiffness measurements and curl retention measurements can only be compared within the "Examples" and within the "Further Examples". A comparison of results between "Examples" and "Further Examples" does not make any sense.

## Claims

1. A method for making a copolymer,
wherein the copolymer comprises as monomers, in polymerized form,
45 to 55 % by weight of an unsaturated amide of the following structure 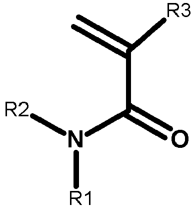 wherein
R1 is selected from the group consisting of H and C1-C8 alkyl,
R2 is selected from the group consisting of H and C1-C8 alkyl,
R3 is selected from the group consisting of H and C1-C8 alkyl,
35 - 45 % by weight of an acrylic acid ester of the following structure 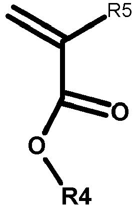 wherein
R4 is selected from the group consisting of C1-C12 alkyl,
R5 is selected from the group consisting of H and C1-C12 alkyl,
9 to 15 % by weight of an acidic monomer comprising a C=C double bond and a COOH group and
0 to 10, preferably 0 to 5, more preferably 0 to 2, % by weight of at least one further monomer having at least one C=C double bond,
wherein the acidic monomer is optionally partially or completely neutralized with a neutralizing agent, wherein the neutralizing agent is preferably selected from the group consisting of triethanolamine, AMP (2-amine-2-jmethyl-1-propanol), sodium hydroxyde, potassium hydroxyde and mixtures thereof,
said method comprising
polymerizing the monomers in a solution comprising the monomers and a solvent, wherein the solvent comprises water and an alcohol selected from the group consisting of ethanol, isopropanol, n-butanol, tert-butanol, pentanol, pentanediol and mixtures thereof.

2. The method of claim 1,
wherein the copolymer comprises as monomers, in polymerized form,
45 to 55 % by weight of t-butylacrylamide,
35 to 45 % by weight of ethyl acrylate,
9 to 15 % by weight of acrylic acid (calculated as free acrylic acid), and
0 to 10, preferably 0 to 5, more preferably 0 to 2, % by weight of at least one further monomer having at least one C=C double bond,
wherein the acrylic acid is optionally partially or completely neutralized with a neutralizing agent, wherein the neutralizing agent is preferably selected from the group consisting of triethanolamine, AMP (2-amine-2-jmethyl-1-propanol), sodium hydroxyde, potassium hydroxyde and mixtures thereof.

3. The method of claim 1
wherein the copolymer comprises as monomers, in polymerized form,
46 to 54 % by weight of t-butylacrylamide,
36 to 44 % by weight of ethyl acrylate,
9 to 14 % by weight of acrylic acid (calculated as free acrylic acid), and
0 to 10, preferably 0 to 5, more preferably 0 to 2, % by weight of at least one further monomer having at least one C=C double bond.

4. The method of claim 1,
wherein the copolymer comprises as monomers, in polymerized form,
47 to 54 % by weight of t-butylacrylamide,
37 to 43 % by weight of ethyl acrylate,
9 to 13 % by weight of acrylic acid (calculated as free acrylic acid), and
0 to 10, preferably 0 to 5, more preferably 0 to 2, % by weight of at least one further monomer having at least one C=C double bond.

5. The method of claim 1,
wherein the copolymer comprises as monomers, in polymerized form,
48 to 53 % by weight of t-butylacrylamide,
37 to 42 % by weight of ethyl acrylate,
9 to 12 % by weight of acrylic acid (calculated as free acrylic acid), and
0 to 10, preferably 0 to 5, more preferably 0 to 2, % by weight of at least one further monomer having at least one C=C double bond.

6. The method of claim 1,
wherein the copolymer comprises as monomers, in polymerized form,
48 to 53 % by weight of t-butylacrylamide,
37 to 41 % by weight of ethyl acrylate,
10 to 12 % by weight of acrylic acid (calculated as free acrylic acid), and
0 to 10, preferably 0 to 5, more preferably 0 to 2, % by weight of at least one further monomer having at least one C=C double bond.

7. The method according to any of claims 1 to 6
wherein the solvent comprises
20 to 70, preferably 20 to 50, more preferably 25 to 35 % by weight of water, and
30 to 80, preferably 50 to 80, more preferably 65 to 75 % by weight of an alcohol selected from the group consisting of ethanol, isopropanol, n-butanol, tert-butanol, pentanol, pentanediol and mixtures thereof.

8. The method according to claim 7, wherein the alcohol is ethanol.

9. A copolymer obtainable by the method according to any of claims 1 to 8.

10. The use of the copolymer according to claim 9 for styling or for fixing human hair.

11. The use of the copolymer according to claim 9 for styling or for fixing human hair, and for providing high gloss to the hair that is styled or fixed.

## Patentansprüche

1. Verfahren zur Herstellung eines Copolymers, wobei das Copolymer als Monomere in polymerisierter Form
45 bis 55 Gew.-% eines ungesättigten Amids der folgenden Struktur 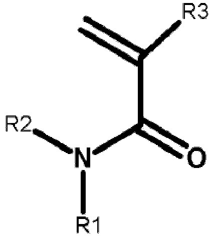 wobei
R1 aus der Gruppe bestehend aus H und C1-C8-Alkyl ausgewählt ist,
R2 aus der Gruppe bestehend aus H und C1-C8-Alkyl ausgewählt ist,
R3 aus der Gruppe bestehend aus H und C1-C8-Alkyl ausgewählt ist,
35-45 Gew.-% eines Acrylsäureesters der folgenden Struktur 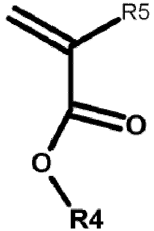 wobei
R4 aus der Gruppe bestehend aus C1-C12-Alkyl ausgewählt ist,
R5 aus der Gruppe bestehend aus H und C1-C12-Alkyl ausgewählt ist,
9 bis 15 Gew.-% eines sauren Monomers mit einer C=C-Doppelbindung und einer COOH-Gruppe und 0 bis 10 Gew.-%, vorzugsweise 0 bis 5 Gew.-%, weiter bevorzugt 0 bis 2 Gew.-%, mindestens eines weiteren Monomers mit mindestens einer C=C-Doppelbindung
umfasst,
wobei das saure Monomer gegebenenfalls teilweise oder vollständig mit einem Neutralisationsmittel neutralisiert ist, wobei das Neutralisationsmittel vorzugsweise aus der Gruppe bestehend aus Triethanolamin, AMP (2-Amino-2-methyl-1-propanol), Natriumhydroxid, Kaliumhydroxid und Mischungen davon ausgewählt ist,
wobei das Verfahren Folgendes umfasst:
Polymerisieren der Monomere in einer Lösung, die die Monomere und ein Lösungsmittel umfasst, wobei das Lösungsmittel Wasser und einen Alkohol aus der Gruppe bestehend aus Ethanol, Isopropanol, n-Butanol, tert-Butanol, Pentanol, Pentandiol und Mischungen davon umfasst.

2. Verfahren nach Anspruch 1,
wobei das Copolymer als Monomere in polymerisierter Form
45 bis 55 Gew.-% t-Butylacrylamid,
35 bis 45 Gew.-% Ethylacrylat,
9 bis 15 Gew.-% Acrylsäure (berechnet als freie Acrylsäure) und
0 bis 10 Gew.-%, vorzugweise 0 bis 5 Gew.-%, weiter bevorzugt 0 bis 2 Gew.-%, mindestens eines weiteren Monomers mit mindestens einer C=C-Doppelbindung
umfasst,
wobei die Acrylsäure gegebenenfalls teilweise oder vollständig mit einem Neutralisationsmittel neutralisiert ist, wobei das Neutralisationsmittel vorzugsweise aus der Gruppe bestehend aus Triethanolamin, AMP (2-Amino-2-methyl-1-propanol), Natriumhydroxid, Kaliumhydroxid und Mischungen davon ausgewählt ist.

3. Verfahren nach Anspruch 1,
wobei das Copolymer als Monomere in polymerisierter Form
46 bis 54 Gew.-% t-Butylacrylamid,
36 bis 44 Gew.-% Ethylacrylat,
9 bis 14 Gew.-% Acrylsäure (berechnet als freie Acrylsäure) und
0 bis 10 Gew.-%, vorzugweise 0 bis 5 Gew.-%, weiter bevorzugt 0 bis 2 Gew.-%, mindestens eines weiteren Monomers mit mindestens einer C=C-Doppelbindung
umfasst.

4. Verfahren nach Anspruch 1,
wobei das Copolymer als Monomere in polymerisierter Form
47 bis 54 Gew.-% t-Butylacrylamid,
37 bis 43 Gew.-% Ethylacrylat,
9 bis 13 Gew.-% Acrylsäure (berechnet als freie Acrylsäure) und
0 bis 10 Gew.-%, vorzugweise 0 bis 5 Gew.-%, weiter bevorzugt 0 bis 2 Gew.-%, mindestens eines weiteren Monomers mit mindestens einer C=C-Doppelbindung
umfasst.

5. Verfahren nach Anspruch 1,
wobei das Copolymer als Monomere in polymerisierter Form
48 bis 53 Gew.-% t-Butylacrylamid,
37 bis 42 Gew.-% Ethylacrylat,
9 bis 12 Gew.-% Acrylsäure (berechnet als freie Acrylsäure) und
0 bis 10 Gew.-%, vorzugweise 0 bis 5 Gew.-%, weiter bevorzugt 0 bis 2 Gew.-%, mindestens eines weiteren Monomers mit mindestens einer C=C-Doppelbindung
umfasst.

6. Verfahren nach Anspruch 1,
wobei das Copolymer als Monomere in polymerisierter Form
48 bis 53 Gew.-% t-Butylacrylamid,
37 bis 41 Gew.-% Ethylacrylat,
10 bis 12 Gew.-% Acrylsäure (berechnet als freie Acrylsäure) und
0 bis 10 Gew.-%, vorzugweise 0 bis 5 Gew.-%, weiter bevorzugt 0 bis 2 Gew.-%, mindestens eines weiteren Monomers mit mindestens einer C=C-Doppelbindung
umfasst.

7. Verfahren nach einem der Ansprüche 1 bis 6,
wobei das Lösungsmittel
20 bis 70 Gew.-%, vorzugsweise 20 bis 50 Gew.-%, weiter bevorzugt 25 bis 35 Gew.-%, Wasser und
30 bis 80 Gew.-%, vorzugsweise 50 bis 80 Gew.-%, weiter bevorzugt 65 bis 75 Gew.-%, eines Alkohols aus der Gruppe bestehend aus Ethanol, Isopropanol, n-Butanol, tert-Butanol, Pentanol, Pentandiol und Mischungen davon
umfasst.

8. Verfahren nach Anspruch 7, wobei es sich bei dem Alkohol um Ethanol handelt.

9. Copolymer, das durch das Verfahren nach einem der Ansprüche 1 bis 8 erhältlich ist.

10. Verwendung des Copolymers nach Anspruch 9 zur Gestaltung oder zur Fixierung von menschlichem Haar.

11. Verwendung des Copolymers nach Anspruch 9 zur Gestaltung oder zur Fixierung von menschlichem Haar und zum Versehen des gestalteten bzw. fixierten Haars mit hohem Glanz.

## Revendications

1. Méthode de préparation d'un copolymère,
dans laquelle le copolymère comprend, comme monomères, sous forme polymérisée,
de 45 à 55% en poids d'un amide insaturé de structure suivante 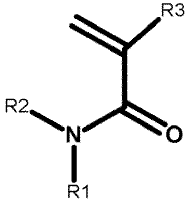 où
R1 est choisi dans le groupe constitué par H et C₁-C₈-alkyle ;
R2 est choisi dans le groupe constitué par H et C₁-C₈-alkyle ;
R3 est choisi dans le groupe constitué par H et C₁-C₈-alkyle ;
35-45% en poids d'un ester d'acide acrylique de structure suivante 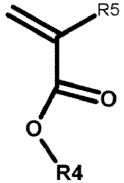 où
R4 est choisi dans le groupe constitué par C₁-C₁₂-alkyle ;
R5 est choisi dans le groupe constitué par H et C₁-C₁₂-alkyle ;
de 9 à 15% en poids d'un monomère acide comprenant une double liaison C=C et un groupement COOH ; et
de 0 à 10, préférablement de 0 à 5, plus préférablement de 0 à 2% en poids d'au moins un autre monomère ayant au moins une double liaison C=C ;
où le monomère acide est éventuellement partiellement ou complètement neutralisé par un agent de neutralisation, où l'agent de neutralisation est choisi de préférence dans le groupe constitué par la triéthanolamine, l'AMP (2-amino-2-méthyl-1-propanol), l'hydroxyde de sodium, l'hydroxyde de potassium et des mélanges de ceux-ci, ladite méthode comprenant
la polymérisation des monomères dans une solution comprenant les monomères et un solvant, où le solvant comprend de l'eau et un alcool choisi dans le groupe constitué par l'éthanol, l'isopropanol, le n-butanol, le tertio-butanol, le pentanol, le pentanediol et des mélanges de ceux-ci.

2. Méthode selon la revendication 1, dans laquelle le copolymère comprend comme monomères, sous forme polymérisée,
de 45 à 55% en poids de t-butylacrylamide,
de 35 à 45% en poids d'acrylate d'éthyle,
de 9 à 15% en poids d'acide acrylique (calculé sous forme d'acide acrylique libre), et
de 0 à 10, préférablement de 0 à 5, plus préférablement de 0 à 2% en poids d'au moins un autre monomère ayant au moins une double liaison C=C,
où l'acide acrylique est éventuellement partiellement ou complètement neutralisé par un agent de neutralisation, où l'agent de neutralisation est choisi de préférence dans le groupe constitué par la triéthanolamine, l'AMP (2-amino-2-méthyl-1-propanol), l'hydroxyde de sodium, l'hydroxyde de potassium et des mélanges de ceux-ci.

3. Méthode selon la revendication 1, dans laquelle le copolymère comprend comme monomères, sous forme polymérisée,
de 46 à 54% en poids de t-butylacrylamide,
de 36 à 44% en poids d'acrylate d'éthyle,
de 9 à 14% en poids d'acide acrylique (calculé sous forme d'acide acrylique libre), et
de 0 à 10, préférablement de 0 à 5, plus préférablement de 0 à 2% en poids d'au moins un autre monomère ayant au moins une double liaison C=C.

4. Méthode selon la revendication 1, dans laquelle le copolymère comprend comme monomères, sous forme polymérisée,
de 47 à 54% en poids de t-butylacrylamide,
de 37 à 43% en poids d'acrylate d'éthyle,
de 9 à 13% en poids d'acide acrylique (calculé sous forme d'acide acrylique libre), et
de 0 à 10, préférablement de 0 à 5, plus préférablement de 0 à 2% en poids d'au moins un autre monomère ayant au moins une double liaison C=C.

5. Méthode selon la revendication 1, dans laquelle le copolymère comprend comme monomères, sous forme polymérisée,
de 48 à 53% en poids de t-butylacrylamide,
de 37 à 42% en poids d'acrylate d'éthyle,
de 9 à 12% en poids d'acide acrylique (calculé sous forme d'acide acrylique libre), et
de 0 à 10, préférablement de 0 à 5, plus préférablement de 0 à 2% en poids d'au moins un autre monomère ayant au moins une double liaison C=C.

6. Méthode selon la revendication 1, dans laquelle le copolymère comprend comme monomères, sous forme polymérisée,
de 48 à 53% en poids de t-butylacrylamide,
de 37 à 41% en poids d'acrylate d'éthyle,
de 10 à 12% en poids d'acide acrylique (calculé sous forme d'acide acrylique libre), et
de 0 à 10, préférablement de 0 à 5, plus préférablement de 0 à 2% en poids d'au moins un autre monomère ayant au moins une double liaison C=C.

7. Méthode selon l'une quelconque des revendications 1 à 6, dans laquelle le solvant comprend
de 20 à 70, préférablement de 20 à 50, plus préférablement de 25 à 35% en poids d'eau, et
de 30 à 80, préférablement de 50 à 80, plus préférablement de 65 à 75% en poids d'un alcool choisi dans le groupe constitué par l'éthanol, l'isopropanol, le n-butanol, le tertio-butanol, le pentanol, le pentanediol et des mélanges de ceux-ci.

8. Méthode selon la revendication 7, dans laquelle l'alcool est l'éthanol.

9. Copolymère pouvant être obtenu par la méthode selon l'une quelconque des revendications 1 à 8.

10. Utilisation du copolymère selon la revendication 9, pour le coiffage ou pour la fixation des cheveux humains.

11. Utilisation du copolymère selon la revendication 9, pour le coiffage ou pour la fixation des cheveux humains, et pour conférer une brillance élevée aux cheveux qui sont coiffés ou fixés.
